# EUROPÄISCHE PATENTSCHRIFT

(11) **EP 2 948 496 B1**
(45) Veröffentlichungstag und Bekanntmachung des Hinweises auf die Patenterteilung: **12.06.2019**
(21) Anmeldenummer: 14712557.9
(22) Anmeldetag: 22.01.2014
(51) Int. Cl.: C08G 73/02

(54) **NEUE BIOAKTIVE POLYMERE**
NEW BIOACTIVE POLYMERS
NOUVEAUX POLYMÈRES BIOACTIFS

(30) Priorität: 25.01.2013 AT 532013
(43) Veröffentlichungstag der Anmeldung: 02.12.2015
(73) Patentinhaber: Sealife Pharma GmbH, 3430 Tulln (AT)
(72) Erfinder: PRETSCH, Alexander, A-3524 Gainbrunn (AT); NAGL, Michael, A-1020 Wien (AT); WIESNER, Christoph, A-1160 Wien (AT); BURGMANN, Heinz, A-1140 Wien (AT)
(74) Vertreter: Ellmeyer, Wolfgang
(86) Internationale Anmeldenummer: PCT/AT2014/050026
(87) Internationale Veröffentlichungsnummer: WO 2014/113835

(56) Entgegenhaltungen:
- CN-A- 103 145 981
- GB-A- 1 095 902
- US-A1- 2011 269 936

## Beschreibung

Die vorliegende Erfindung betrifft neue bioaktive Polymere, sowie deren Verwendung als Biozide.

### STAND DER TECHNIK

Polyguanidine der nachstehenden allgemeinen Formel wie auch diverse Derivate davon sind seit langem bekannt.

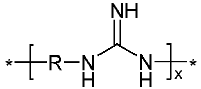

In der Patentliteratur wurden bereits 1943 im US-Patent 2.325.586 mehrere Herstellungsverfahren verschiedener Polyguanidine durch Polykondensation von i) Guanidin oder Salzen davon, ii) Cyanhalogeniden, iii) Dicyanamiden oder iv) Isocyaniddihalogeniden mit Diaminen oder v) zweier Dicyandiamide miteinander (was Cyano-substituierte Polyguanidine ergibt), sowie die Verwendung der so erzeugten Polyguanidine als Färbehilfsmittel beschrieben:
i) 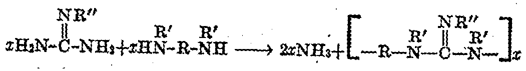
ii) 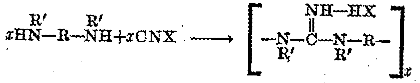
iii) 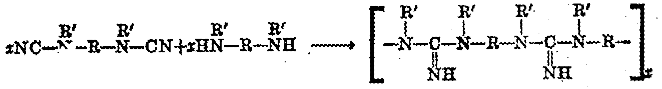
iv) 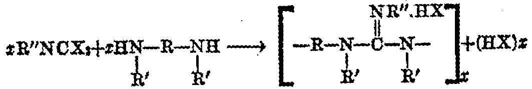
v)

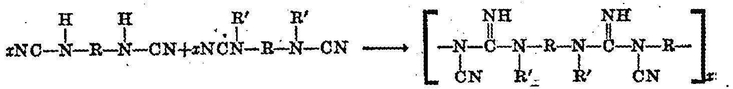

Als Diamine in den Reaktionen i) bis iv) wurden bereits damals sowohl Alkylen- und Phenylendiamine als auch Oxyalkylen- oder Polyetherdiamine, wie sie später auch als Jeffamine® bekannt werden sollten, offenbart.

Jahrzehnte später haben sich derartige Polyguanidine auch als ausgezeichnete Biozide erwiesen. So offenbart eine Gruppe um Oskar Schmidt in WO 99/54291 A1 die Herstellung von mikrobioziden Polyhexamethylenguanidinen, in WO 01/85676 A1 biozide Polyguanidine, die durch Kondensation von Guanidin mit Polyoxyalkylenen hergestellt werden, und in WO 2006/047800 A1 als Biozide, insbesondere als Fungizide, wirkende Polyguanidinderivate, die durch Polykondensation von Guanidin mit einem Gemisch aus Alkylendiamin und Oxalkylendiamin gebildet werden und geringere Toxizität aufweisen sollen als die Polymere, die nur eine der beiden Arten des zweiwertigen Rests R₁ enthalten.

In WO 02/30877 A1 werden ähnliche Polyguanidine als Desinfektionsmittel beschrieben, die zusätzlich Phenylengruppierungen in den Ketten enthalten. Eine russische Forschergruppe (Tets, Tets und Krasnov) offenbart in WO 2011/043690 A1, von der US 2011/0269936 A1 und EP 2.520.605 A1 abgeleitet wurden, biozide Polyguanidine der nachstehenden Formel, die durch Polykondensation von Guanidin und Hexamethylendiamin in Gegenwart von Hydrazinhydrat hergestellt werden:

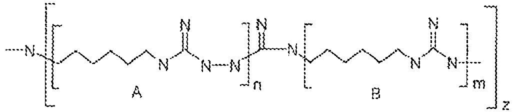

Das Hydrazin ersetzt somit während der Polykondensation - zumindest formal - eine Aminogruppe entweder nur einer oder auch zweier Guanidin-Gruppierungen, wodurch Block-Copolymere erhalten werden sollen, in denen sich Poly(hexamethylen-guanidin)-Blöcke mit Poly(hexamethylenaminoguanidin)-Blöcken abwechseln und die beiden Arten von Blöcken jeweils über Guanidin-Dimere miteinander verknüpft sind, wie nachstehend dargestellt ist:

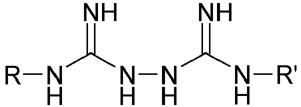

Auch diese Polymere und Säureadditionssalze davon sollen als Biozide gegen Bakterien, Viren und Pilze wirken. Allerdings finden sich in den Beispielen dieser Anmeldungen, in denen 7 verschiedene Polymere hergestellt wurden, außer der Angabe, dass jenes aus Beispiel 1 eine "feste, nahezu farblose, transparente Substanz" sei, keinerlei physikalische Daten zu den erhaltenen Produkten.

Bezüglich der möglichen Strukturen, die während der Polykondensationen von Guanidinen mit Diaminen entstehen können, existieren mehrere Artikel einer Forschergruppe der Technischen Universität Graz, z.B. Albert et al., Biomacromolecules 4(6), 1811-1817 (2003), und Feiertag et al., Macromol. Rap. Comm. 24(9), 567-570 (2003). Zusätzlich zu den verschiedenen Möglichkeiten der Terminierung der linearen Polymerketten mit jeweils einem der Ausgangsmonomere bilden sich üblicherweise in einem nicht zu vernachlässigenden Anteil, der u.a. von der Kettenlänge des Diamins abhängt, auch zyklische Moleküle der folgenden allgemeinen Formel aus:

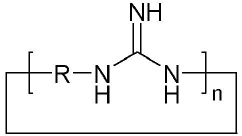

Die Hauptnachteile praktisch aller der oben beschriebenen Polyguanidin-Derivate liegen zum einen in einer nicht zu vernachlässigenden Toxizität dieser Produkte sowie - im Fall des Einsatzes hochreaktiver Komponenten - in vergleichsweise aufwändigen Herstellungsverfahren, sowie auch in der Verwendung von aus toxikologischer Sicht bekanntermaßen problematischen Komponenten wie Hydrazin, weswegen das Ziel der Erfindung darin bestand, neue, weniger toxische, aber weiterhin als Biozide wirksame Polyguanidine herzustellen, und das auf möglichst einfache und wirtschaftliche Weise unter Umgehung der oben genannten Nachteile.

### OFFENBARUNG DER ERFINDUNG

Dieses Ziel erreicht die vorliegende Erfindung durch Bereitstellung neuer Polykondensationsprodukte von Aminoguanidin und/oder 1,3-Diaminoguanidin mit einem oder mehreren Diaminen, wobei die Polykondensationsprodukte Polyguanidin-Derivate der nachstehenden Formel (I) sind: 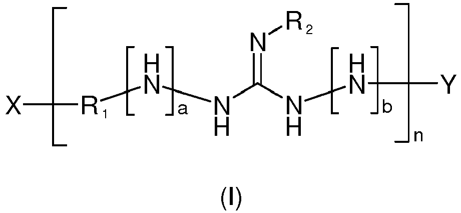 worin
X aus -NH₂, Aminoguanidino und 1,3-Diaminoguanidino ausgewählt ist;
Y aus -H und -R₁-NH₂ ausgewählt ist;
oder X und Y zusammen für eine chemische Bindung stehen, um eine zyklische Struktur zu ergeben;
R₁ aus zweiwertigen organischen Resten mit 2 bis 20 Kohlenstoffatomen ausgewählt ist, in denen gegebenenfalls ein oder mehrere Kohlenstoffatome durch O oder N ersetzt sind;
a und b jeweils 0 oder 1 sind,
   wobei a+b ≠ 2 ist, wenn keine 1,3-Diaminoguanidin-Einheiten enthalten sind;
R₂ aus -H und -NH₂ ausgewählt ist, wobei
   R₂ -NH₂ ist, wenn a+b = 0 ist,
   R₂ -H oder -NH₂ ist, wenn a+b = 1 ist und
   R₂ -H ist, wenn a+b = 2 ist; und
n ≥ 2 ist;
oder von Salzen davon.

Die neuen Polyguanidin-Derivate der Formel (I) haben sich in Aktivitätstests als wirksame antimikrobielle Substanzen erwiesen, die jedoch überraschenderweise deutlich geringere Toxizität als die strukturell ähnlichen Polymere aus den oben zitierten Dokumenten WO 2011/043690 A1, US 2011/0269936 A1 und EP 2.520.605 A1 zeigen, wie dies durch die späteren Ausführungsbeispiele der Erfindung und Vergleichsbeispiele belegt wird. Ohne sich auf eine Theorie einschränken zu wollen, nehmen die Erfinder an, dass Amino- und Diaminoguanidino-Gruppierungen für humane eukaryotische Zellen besser verträglich sind als Guanidino-Gruppierungen und insbesondere als jene Polymere, die die oben dargestellten hydrazoverbrückten Guanidin-Dimere enthalten. Darüber hinaus wird im offenbarten Verfahren die Verwendung der toxischen Komponente Hydrazinhydrat im Polymerisationsprozess vermieden, das in manchen Polymeren nach dem Stand der Technik als Restmonomer enthalten sein kann.

Die obige Formel (I) stellt sowohl Polykondensationsprodukte von (Mono-)Aminoguanidin, nachstehend kurz als MAG bezeichnet, als auch solche von 1,3-Diaminoguanidin, nachstehend kurz als DAG bezeichnet, dar.

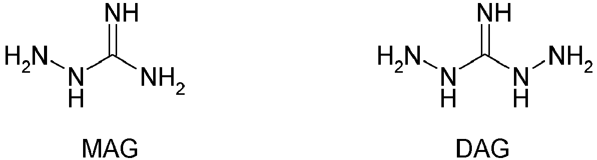

Formel (I) erklärt sich dadurch, dass während der unter Ammoniakabspaltung ablaufenden Polykondensation die MAG- und DAG-Reste sowohl über ihre Amino- oder tautomere Imino-Gruppe als auch über ihre Hydrazo- (Hydrazinyl-) Gruppierung(en) an der Polykondensation teilnehmen können. Folglich existieren sowohl für MAG als auch für DAG als Ausgangsmonomer drei verschiedene Möglichkeiten des Einbaus in die Ketten der erfindungsgemäßen Polymere. Im Fall von MAG kann in Formel (I) die einzige Hydrazo-Gruppierung, im Fall von DAG hingegen die einzige Imino-/Amino-Gruppierung nach links, nach rechts oder nach oben weisen.

Dies bedeutet für MAG folgende mögliche Parameter in Formel (I):

| | |
|---|---|
| a = 1, b = 0, R₂ ist -H: | Hydrazo-Gruppierung weist nach links; |
| a = 0, b = 1, R₂ ist -H: | Hydrazo-Gruppierung weist nach rechts; oder |
| a = 0, b = 0, R₂ ist -NH₂: | Hydrazo-Gruppierung weist nach oben. |

Für DAG gibt es folgende mögliche Parameterkombinationen:

| | |
|---|---|
| a = 0, b = 1, R₂ ist -NH₂: | Amino-/Imino-Gruppierung weist nach links; |
| a = 1, b = 0, R₂ ist -NH₂: | Amino-/Imino-Gruppierung weist nach rechts; oder |
| a = 1, b = 1, R₂ ist -H: | Amino-/Imino-Gruppierung weist nach oben. |

Ohne sich darauf beschränken zu wollen, scheinen NMR-Spektren der erhaltenen Polykondensate, wie in den späteren Beispielen für die Erfindung beschrieben, zu belegen, dass während der Polykondensationsreaktionen durchwegs Gemische mehrerer der drei Ausrichtungsmöglichkeiten erhalten wurden, wobei davon auszugehen ist (was bislang noch nicht 100%ig abgeklärt wurde), dass auch innerhalb einer Kette mehrere Ausrichtungen ein und desselben Monomers vorkommen.

Im obigen Zusammenhang sei weiters explizit darauf hingewiesen, dass die Lage der C=N-Doppelbindungen der Guanidino-Gruppierungen - wie auch die räumliche Stellung des Substituenten R₂ an der Doppelbindung - den üblichen Tautomerieeffekten unterliegen. Das heißt, dass die Doppelbindung des Guanidins innerhalb oder außerhalb der Kette liegen kann und R₂ nach links oder rechts weisen kann. Solche Tautomere der obigen Polykondensationsprodukte der Formel (I) sollen daher ebenfalls im Schutzumfang der vorliegenden Erfindung liegen.

Die obigen Optionen für X und Y ergeben sich durch die unterschiedlichen Möglichkeiten der Kettenterminierung - je nachdem, ob MAG, DAG oder ein Gemisch der beiden als Ausgangsmonomer eingesetzt wurde - einschließlich der Möglichkeit des Ringschlusses zu einem zyklischen Polykondensat. Siehe hierzu auch die zuvor zitierten Artikel von Albert et al. und von Feiertag et al. Für endständige Aminoguanidino- (MAG-) und 1,3-Diaminoguanidino- (DAG-) Gruppierungen gelten natürlich dieselben Optionen wie für innerhalb der Ketten liegende, d.h. dass die Anbindung an die Kette über ein beliebiges Stickstoffatom gegeben sein kann.

Der Rest R₁ kann gemäß vorliegender Erfindung ein linearer, verzweigter oder zyklischer, gesättigter oder ungesättigter, zweiwertiger Kohlenwasserstoff-Rest mit 2 bis 20 Kohlenstoffatomen, vorzugsweise 4 bis 18 Kohlenstoffatomen, noch bevorzugter 6 bis 12 Kohlenstoffatomen, sein, in dem manche C-Atome durch O und/oder N ersetzt sein können. Die obigen Bevorzugungen ergaben sich aus folgenden Überlegungen. Bei sehr kurzen Resten R₁ sind die aktiven MAG- bzw. DAG-Gruppierungen sehr nahe beieinander, was die Aktivität der Polymere verringern kann, bei längeren Resten hingegen recht weit auseinander. Reste mit mehr als 20 Atomen kommen daher prinzipiell zwar auch in Frage, sind aber vom wirtschaftlichen Standpunkt aus gesehen nicht zu bevorzugen, da sie Polymere der Formel (I) ergeben, in denen pro Gewichtseinheit nur relativ wenige antiinfektiv wirksame Guanidino-Gruppierungen enthalten sind.

Vorzugsweise ist der Rest R₁ aus Alkylenresten ausgewählt, in denen gegebenenfalls ein oder mehrere Kohlenstoffatome durch O oder N ersetzt sind, was die Hydrophilie der Ketten erhöht, noch bevorzugter aus Resten der nachstehenden allgemeinen Formeln (II) bis (V):

-(CH₂)_{c}-Z₁-(CH₂)_{d}-, (II)

-(CH₂)_{c}-Z₁-(CH₂)_{d}-Z₂-(CH₂)ₑ-, (III)

-(CH₂)_{c}-Z₁-(CH₂)_{d}-Z₂-(CH₂)ₑ-Z₃-(CH₂)_{f}-, (IV)

-(CH₂)_{c}-Z₁-(CH₂)_{d}-Z₂-(CH₂)ₑ-Z₃-(CH₂)_{f}-Z₄-(CH₂)_{g}-, (V)

worin Z₁ bis Z₄ jeweils unabhängig voneinander für ein aus O und N ausgewähltes Heteroatom stehen und die Indizes c bis g jeweils unabhängig voneinander für eine solche ganze Zahl im Bereich von 1 bis 12 stehen, dass die Gesamtanzahl der Atome des Rests R₁ 20 nicht überschreitet. Besonders bevorzugt wird, dass innerhalb eines Rests R₁ alle Heteroatome Z entweder O oder N sind.

Die besten Ergebnisse bei Tests auf ihre Biozid-Wirkung bzw. Toxizität wurden mit Verbindungen erzielt, in denen R₁ für den zweiwertigen Rest eines Polyetherdiamins, wie z.B. 4,9-Dioxadodecan-1,12-diamin, eines Polyoxyethylen- und/oder -propylendiamins, wobei n vorzugsweise für 2 bis 15, noch bevorzugter 2 bis 10, insbesondere 2 bis 6, steht.

Als Salze der neuen Polyguanidine der Formel (I) kommen jegliche Säureadditionssalze mit einer oder mehreren anorganischen oder organischen Säuren in Frage, wie z.B. Halogenwasserstoffsäuren, Sauerstoffsäuren von Stickstoff, Schwefel oder Phosphor, Borsäure, Kohlensäure, Carbon-, Thiocarbon-, Carbamin-, Sulfon-, Phosphon- oder Phosphinsäuren, sowie partielle Ester oder Amide mehrwertiger Vertreter dieser Säuren. Bevorzugt werden gemäß vorliegender Erfindung pharmazeutisch annehmbare Salze eingesetzt, noch bevorzugter Säure-Additionssalze als Hydrochlorid, Hydrobromid, Hydroiodid, Sulfat, Methylsulfat, Carbonat, Borat, Cyanat, Thiocyanat, Phosphat, Mesylat, Nitrat, Acetat, Benzoat, Lactat, Tartrat, Citrat, Maleat, Fumarat oder Partialestern dieser Säuren, sofern sie di- oder höherfunktionell sind. Als Alkoholkomponente solcher Partialester wird ein pharmazeutisch annehmbarer Alkohol bevorzugt, der insbesondere Ethanol ist

Enthält der Rest R₁ eine oder mehrere OH- oder COOH-Gruppen, so fallen auch Salze mit anorganischen oder organischen Basen in den Schutzumfang der vorliegenden Erfindung, vorzugsweise mit pharmazeutisch annehmbaren Basen, noch bevorzugter mit einem Guanidinderivat, insbesondere mit Amino- oder Diaminoguanidin, d.h. mit dem Guanidinderivat, von dem bei der Herstellung der erfindungsgemäßen neuen Polyguanidine ausgegangen wird. In der Regel wird sich bei der Herstellung ohnehin ein inneres Salz aus sauren und basischen Gruppierungen innerhalb der jeweiligen Moleküle ausbilden.

In einem zweiten Aspekt der Erfindung wird ein Verfahren zur Herstellung von erfindungsgemäßen Polyguanidin-Derivaten nach dem ersten Aspekt durch Polykondensation eines Guanidin-Derivats oder eines Salzes davon mit einem Diamin bereitgestellt, das dadurch gekennzeichnet ist, dass MAG und/oder DAG oder ein Säureadditionssalz davon mit zumindest einem Diamin H₂N-R₁-NH₂ durch Erhitzen polykondensiert wird.

Im Gegensatz zum Stand der Technik umfasst das Verfahren der Erfindung die Reaktion von MAG oder DAG mit einem oder mehreren Diaminen, vorzugsweise nur einem einzigen Diamin. Dadurch können eindeutiger definierte Produkte hergestellt werden als etwa bei den oben zitierten Arbeiten der russischen Forscher, zumal in den erfindungsgemäß hergestellten Reaktionsmischungen freies Hydrazin in keiner Phase des Reaktionsverlaufs nachweisbar war - weder chromatographisch noch nasschemisch. (Neben-)Reaktionen mit Hydrazin, die im angesprochenen Stand der Technik zwar erwünscht waren, hierin jedoch gänzlich unerwünscht sind, konnten somit wirksam vermieden werden.

Vorzugsweise wird das Verfahren der Erfindung durchgeführt, indem ein Salz von MAG oder DAG, insbesondere deren Hydrochlorid, zusammen mit dem Diamin, das vorzugsweise in einem geringfügigen molaren Überschuss, z.B. von 3 bis 5 Mol-% oder, aus Gründen der Wirtschaftlichkeit, maximal 10 Mol-%, bezogen auf (Di-)Aminoguanidin, eingesetzt wird, um vollständigen Umsatz des Guanidinderivats zu gewährleisten, zunächst auf eine erste, niedrigere Temperatur, vorzugsweise etwa 80-150 °C, noch bevorzugter 110-130 °C, und dann auf eine zweite, höhere Temperatur, vorzugsweise 150-250 °C, noch bevorzugter 160-180 °C, erhitzt wird, um die Reaktionsgeschwindigkeit und damit auch die Gasentwicklung zu steuern. Das Reaktionsgemisch wird dabei vorzugsweise 1 bis 3 h, noch bevorzugter etwa 2 h, lang auf der ersten Temperatur und dann vorzugsweise 1 bis 8 h, noch bevorzugter 3 bis 5 h, lang auf der zweiten Temperatur gehalten, um vollständige Reaktion zu gewährleisten.

Die Reaktion wird vorzugsweise bei Normaldruck sowie unter Wasserausschluss durchgeführt, was beispielsweise durch zumindest anfängliches Spülen des Reaktionsgefäßes mit Inertgas und Vorsehen eines Trockenrohrs am Reaktionsgefäß bewirkt werden kann. Es kann allerdings auch ein Vakuum angelegt werden, vor allem gegen Ende der Reaktion in Form eines Reinigungsschrittes, um freien Ammoniak sowie Restmonomere, d.h. vor allem Überschüsse des Diamins, möglichst vollständig abzudampfen.

Nach Abschluss der Reaktion wird das erhaltene Polyguanidin-Derivat vorzugsweise in Wasser, z.B. in etwa der 3- bis 10fachen Menge Wasser, gelöst. Dies dient einerseits der Abtrennung etwaiger wasserunlöslicher Komponenten, und andererseits stellt eine wässrige Lösung eine bevorzugte Formulierung für die Anwendung der neuen Polymere dar, ist also - gegebenenfalls nach Zusatz optionaler Adjuvantien - mitunter direkt als solche einsetzbar.

Weitere, derzeit weniger bevorzugte Möglichkeiten der Reinigung bestünden beispielsweise im Abdampfen des Wassers aus der wässrigen Lösung und Trocknung der Polymere im Vakuum oder im Aussalzen aus der wässrigen Lösung durch Säurezusatz und anschließende Trocknung, wofür die oben erwähnten Säuren, insbesondere die als bevorzugt beschriebenen pharmazeutisch annehmbaren Säuren in Frage kommen. Eine Ausführungsform des Aussalzens umfasst das Einleiten von CO₂ und Aussalzen des Polyguanidins als Carbonat bzw. Hydrogencarbonat. Falls das gewünschte Polyuganidin nicht als Salz, sondern als freie Base zum Einsatz kommen soll, kann auf das Aussalzen eine Behandlung mit einer Base folgen, die in wässriger oder auch nichtwässriger Lösung oder Suspension vorliegen kann.

In einem dritten Aspekt betrifft die Erfindung auch ein Polyguanidin-Derivat gemäß dem ersten Aspekt der Erfindung oder hergestellt durch ein Verfahren gemäß dem zweiten Aspekt der Erfindung zur Verwendung im human- und veterinärmedizinischen Bereich zur Bekämpfung von bakteriellen, fungalen und viralen Infektionen und deren Folgeerscheinungen, als Pestizid und Desinfektionsmittel im Agrar- und Umweltbereich, allgemein als Desinfektionsmittel (Biozid) zur Keimreduktion und -vernichtung, als Antiparasitikum, als Zusatz zur Stabilisierung (Sterilisation) von Produkten oder auch als Vernebelungssubstanz in gelöster Form zur Kalt-/Nass-Vernebelung, Mikronisierung und Dampfsterilisation.

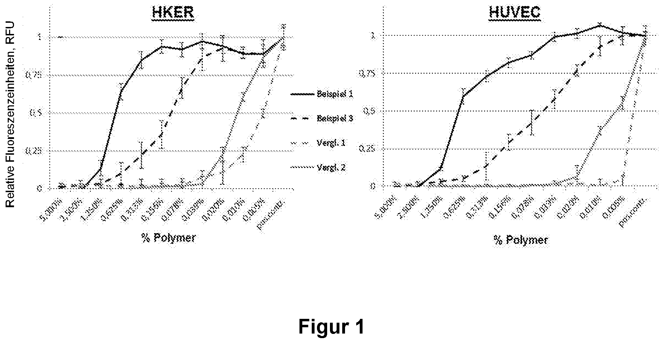

Nachstehend wird die vorliegende Erfindung anhand nichteinschränkender Ausführungsbeispiele zusammen mit Vergleichsbeispielen näher beschrieben. In der einzigen Zeichnung, Fig. 1, sind die Ergebnisse von Toxizitätstests angegeben.

### BEISPIELE

### Beispiele 1 bis 6 & Vergleichsbeispiele 1 und 2 - Herstellung der Polymere

### Beispiel 1

23 mmol 1,3-Diaminoguanidinium-Hydrochlorid und 24 mmol 4,9-Dioxadodecan-1,12-diamin wurden in einem mit einem Trockenrohr verschlossenen Reaktionsgefäß 90 min lang unter Rühren auf 120 °C erhitzt, danach wurde die Temperatur für 100 min auf 180 °C erhöht, davon am Ende dieser Reaktionszeit für 45 min unter vermindertem Druck (50 mbar). Nach Abkühlen der Reaktionsmischung auf unter 80 °C wurden zum gelartigen Reaktionsprodukt 25 ml Wasser zugesetzt. Nach einigen Stunden wurde eine klare Lösung erhalten.

Von einer Probe der erhaltenen wässrigen Lösung wurde das Wasser abgedampft, und der erhaltene Rückstand wurde im Vakuum getrocknet, wobei eine rötliche, viskose Flüssigkeit erhalten wurde. Diese wurde in 2 ml D₂O (mit einem Deuterierungsgrad > 99,5%) gelöst, und ein ¹H-Kernresonanz- (¹H-NMR-) Spektrum wurde aufgenommen. Die Lage der auf diese Weise unterscheidbaren Gruppen von Methylen-Protonen der Reste R₁ im Produkt ist wie folgt:
¹H-NMR (D₂O), δ (ppm): 1,54-1,67 (m, OCH₂*CH₂CH₂*CH₂O), 1,80-1,95 (m, NCH₂*CH₂*), 3,23-3,38 ppm (m, N*CH₂*), 3,42-3,65 ppm (m, CH₂*CH₂*O*CH₂*CH₂).

Dies bestätigt die Struktur der verwendeten Diamin-Komponente, 4,9-Dioxadodecan-1,12-diamin.

### Beispiel 2

4,6 mmol 1,3-Diaminoguanidinium-Hydrochlorid und 4,8 mmol 4,9-Dioxadodecan-1,12-diamin wurden in einem mit einem Trockenrohr verschlossenen Reaktionsgefäß 90 min lang unter Rühren auf 120 °C erhitzt, danach wurde die Temperatur für 8 h auf 180 °C erhöht, davon am Ende dieser Reaktionszeit für 45 min unter vermindertem Druck (50 mbar). Nach Abkühlen der Reaktionsmischung auf unter 80 °C wurden dem gelartigen Reaktionsprodukt 16 ml Wasser zugesetzt. Nach einigen Stunden wurde eine klare Lösung erhalten.

### Beispiel 3

4,6 mmol N-Aminoguanidinium-Hydrochlorid und 4,8 mmol 4,9-Dioxadodecan-1,12-diamin wurden in einem mit einem Trockenrohr verschlossenen Reaktionsgefäß 90 min lang unter Rühren auf 120 °C erhitzt, danach wurde die Temperatur für 3,5 h auf 180 °C erhöht, davon am Ende dieser Reaktionszeit für 60 min unter vermindertem Druck (50 mbar). Nach Abkühlen der Reaktionsmischung auf unter 80 °C wurden dem gelartigen Reaktionsprodukt 16 ml Wasser zugesetzt. Nach einigen Stunden wurde eine klare Lösung erhalten.

### Beispiel 4

1,16 mmol 1,3-Diaminoguanidinium-Hydrochlorid und 1,21 mmol Tris-(2-aminoethyl)-amin wurden in einem mit einem Trockenrohr verschlossenen Reaktionsgefäß 150 min lang unter Rühren auf 120 °C erhitzt, danach wurde die Temperatur für 2,5 h auf 160 °C erhöht, davon am Ende dieser Reaktionszeit für 45 min unter vermindertem Druck (50 mbar). Nach Abkühlen der Reaktionsmischung auf unter 80 °C wurden dem gelartigen Reaktionsprodukt 4 ml Wasser zugesetzt. Nach einigen Stunden wurde eine klare Lösung erhalten.

### Beispiel 5

8,12 mmol 1,3-Diaminoguanidinium-Hydrochlorid und 8,47 mmol Tris-(2-aminoethyl)-amin wurden in einem mit einem Trockenrohr verschlossenen Reaktionsgefäß 120 min lang unter Rühren auf 130 °C erhitzt, danach wurde die Temperatur für 8 h auf 180 °C erhöht, davon am Ende dieser Reaktionszeit für 90 min unter vermindertem Druck (15 mbar). Nach Abkühlen der Reaktionsmischung auf unter 80 °C wurden dem gelartigen Reaktionsprodukt 28 ml Wasser zugesetzt. Nach einigen Stunden wurde eine klare Lösung erhalten.

### Beispiel 6

2,32 mmol 1,3-Diaminoguanidinium-Hydrochlorid und 2,43 mmol 3,6-Dioxaoctan-1,8-diamin wurden in einem mit einem Trockenrohr verschlossenen Reaktionsgefäß 60 min lang unter Rühren auf 120 °C erhitzt, danach wurde die Temperatur für 4 h auf 170 °C erhöht, davon am Ende dieser Reaktionszeit für 60 min unter vermindertem Druck (50 mbar). Nach Abkühlen der Reaktionsmischung auf unter 80 °C wurden dem gelartigen Reaktionsprodukt 7 ml Wasser zugesetzt. Nach einigen Stunden wurde eine klare Lösung erhalten.

### Vergleichsbeispiel 1

23,2 mmol Guanidinium-Hydrochlorid 5,4 mmol 3,6-Dioxaoctan-1,8-diamin und 18,1 mmol 1,6-Diaminohexan wurden in einem mit einem Trockenrohr verschlossenen Reaktionsgefäß 90 min lang unter Rühren auf 120 °C erhitzt, danach wurde die Temperatur für 8 h auf 170 °C erhöht, davon am Ende dieser Reaktionszeit für 90 min unter vermindertem Druck (50 mbar). Nach Abkühlen der Reaktionsmischung auf unter 80 °C wurden dem gelartigen Reaktionsprodukt 60 ml Wasser zugesetzt. Nach einigen Stunden wurde eine klare Lösung erhalten.

Das erhaltene Polymer entspricht strukturell jenen, die in WO 2006/047800 A1 offenbart werden.

### Vergleichsbeispiel 2

2,00 mmol Guanidinium-Hydrochlorid, 1,70 mmol 1,6-Hexamethylendiamin und 0,3 mmol Hydrazin-Hydrat wurden in einem mit einem Trockenrohr verschlossenen Reaktionsgefäß 90 min lang unter Rühren auf 160 °C erhitzt, danach wurde die Temperatur für 3,5 h auf 180 °C erhöht, davon am Ende dieser Reaktionszeit für 60 min unter vermindertem Druck (50 mbar). Nach Abkühlen der Reaktionsmischung auf unter 80 °C wurden dem gelartigen Reaktionsprodukt 4 ml Wasser zugesetzt. Nach einigen Stunden wurde eine klare Lösung erhalten.

Das erhaltene Polymer entspricht strukturell jenen, die in WO 2011/043690 A1 offenbart werden.

### Beispiel 7 - Aktivitätsbestimmung: antimikrobielle/antifungale/antivirale Wirkung

Die Aktivität der neuen Verbindungen wurde in mehrfach durchgeführten Screeningsystemen getestet. Die antibakterielle und antifungale Aktivität wurde mittels MHK-Test untersucht. MHK steht für die "minimale Hemm-Konzentration" (engl.: MIC für "minimal inhibitory concentration") und bezeichnet die niedrigste Konzentration einer Substanz, bei der mit bloßem Auge keine Vermehrung von Mikroorganismen wahrgenommen werden kann. Bestimmt wird die MHK mit einem so genannten Titerverfahren, bei dem die Substanz ausverdünnt und anschließend der Erreger zugefügt wird.

In der Regel wird so die Konzentration eines Antibiotikums bestimmt, die das Wachstum eines Bakterienstammes gerade noch hemmt. Die MHK wird in Mikrogramm pro Milliliter (µg/ml) oder in Vol.-% angegeben, und die Verdünnungen erfolgen in der Regel in log2-Schritten. Hierin wurde eine Ausgangskonzentration von 1 % jeweils auf das Doppelte verdünnt, was folglich Testkonzentrationen von 0,5 %, 0,25 %, 0,125 % usw. ergab. Niedrigere Werte spiegeln demnach bessere Aktivität als Antiinfektivum wider.

Die Tests wurden nach den vom EUCAST (European Committee for Antimicrobial Susceptibility Testing) geforderten Standards und gemäß den AFST- ("Antifungal Susceptibility Testing") Vorschriften der European Society of Clinical Microbiology and Infectious Diseases (ESCMID) durchgeführt.

Das Screeningsystem für Viren ist ein Infektionssystem, bei dem Wirtszellen *in vitro* infiziert werden und die Testsubstanz vor oder nach der Infektion zugesetzt und ihre Aktivität bestimmt wird. Alle diese Tests wurden gemäß den betriebsinternen Standardvorschriften von SeaLife Pharma zum Arzneimittelscreening durchgeführt, wobei analoge Verdünnungsreihen wie im antibakteriellen/antifungalen Test eingesetzt wurden.

In den nachstehenden Tabellen 1 bis 3 sind die Testergebnisse bezüglich der antiinfektiven Wirkung der erfindungsgemäßen neuen Verbindungen aus den Beispielen 1, 3, 4 und 5 gegen einige multiresistente Bakterien und Pilze sowie Viren angegeben. Die Daten sind jeweils Mittelwerte von Mehrfachbestimmungen.

Es ist klar ersichtlich, dass die neuen Verbindungen der Erfindung ausgezeichnete Aktivität sowohl gegen gram-positive als auch gram-negative Erreger zeigen:

**Tabelle 1**

| Ergebnisse der MHK Tests | MRSA | Staphylococcus epidermidis | Streptococcus pneumoniae | Enterococcus faecalis | Propionibacter acne | E. coli | Klebsiella pneumonia | Pseudomonas aeruginosa | Acinetobacter baumanii | Enterobacter doace | Salmonella enterica |
|---|---|---|---|---|---|---|---|---|---|---|---|
| Beispiel 1 | 0,001% | 0,001% | 0,004% | 0,008% | 0,001% | 0,016% | 0,02% | 0,02% | 0,06% | 0,03% | 0,03% |
| Beispiel 3 | 0,001% | 0,001% | 0,001% | 0,008% | 0,001% | 0,02% | 0,02% | 0,02% | 0,06% | 0,2% | 0,03% |
| Beispiel 4 | 0,001% | 0,001% | 0,001% | 0,008% | 0,001% | 0,016% | 0,016% | 0,030% | 0,02% | 0,016% | 0,030% |
| Beispiel 5 | 0,001% | 0,001% | 0,002% | 0,002% | 0,001% | 0,020% | 0,02% | 0,04% | 0,04% | 0,13% | 0,03% |

Sowie gegen Pilze und Hefen:

**Tabelle 2**

| Ergebnisse der MHK Tests | Candida albicans | Candida papillosis | Candida glabrata | Candida kruzei | Aspergillus terreus | Aspergillus fumigatus | Fusarium rosei | Trichophyton sp. | Alternarria alternarria | Microsporum canis | Dermatiacea sp. |
|---|---|---|---|---|---|---|---|---|---|---|---|
| Beispiel 1 | 0,008% | 0,03% | 0,02% | 0,02% | 0,02% | 0,03% | 0,03% | 0,02% | 0,02% | 0,03% | 0,02% |
| Beispiel 3 | 0,02% | 0,02% | 0,02% | 0,02% | 0,03% | 0,03% | 0,03% | 0,02% | 0,02% | 0,02% | 0,02% |
| Beispiel 4 | 0,008% | 0,016% | 0,016% | 0,008% | 0,125% | 0,125% | n.t. | n.t. | n.t. | n.t. | n.t. |
| Beispiel 5 | 0,02% | 0,02% | 0,02% | 0,020% | 0,016% | 0,016% | n.t. | n.t. | n.t. | n.t. | n.t. |

Wie auch gegen Viren:

**Tabelle 3**

| **Ergebnisse der virologischen Tests** | Influenza A und B | Humaner Rhinovirus | Parainfluenza virus | Herpes simplex virus |
|---|---|---|---|---|
| **Beispiel 1** | 0,008% | 0,008% | 0,008% | 0,02% |
| **Beispiel 3** | 0,02% | 0,02% | 0,02% | 0,02% |
| **Beispiel 4** | 0,04% | 0,02% | 0,04% | 0,02% |
| **Beispiel 5** | 0,04% | 0,04% | 0,04% | 0,02% |

Alle getesteten neuen Verbindungen weisen somit sehr gute bis ausgezeichnete Aktivität gegen diverseste Erreger auf - und das bei deutlich niedrigerer Toxizität als die nach dem Stand der Technik bekannten Polyguanidin-Derivate, wie die nachstehenden Toxizitätstests zeigen.

### Beispiel 8 - Toxizitätstests

Mit Hilfe des AlamarBlue®-Assays, wie nachstehend angegeben, wurden 4 Polymere auf ihr toxikologisches Potential (inkludiert Proliferation, Zelltod, Zellmetabolismus) untersucht und der IC₅₀-Wert sowie die nichttoxische Konzentration an primären Keratinozyten (HKER) und primären Endothelzellen (HUVEC) bestimmt. In Fig. 1 ist der toxische Effekt der unterschiedlichen Polymere konzentrationsabhängig dargestellt.

AlamarBlue®-Assay: 20.000 humane Keratinozyten (HKER) bzw. Endothelzellen (HUVEC) wurden in 96-Well-Platten ausplattiert und 24 h lang inkubiert, bevor unterschiedliche Konzentrationen (5 % bis 0,005 %) der neuen Polymere aus den Beispielen 1 und 3 sowie der Vergleichssubstanzen aus den Vergleichsbeispielen 1 und 2 zugesetzt wurden. Nach 24 Stunden wurden in jedes Well (100 µl Medium) 10 µl AlamarBlue® beigemengt, und nach 3-stündiger Inkubation wurde die Farbreaktion mittels Multiplatten-Reader detektiert (Ex: 530 nm; Em: 590 nm). HKER: "human primary keratinocytes", menschliche Primärkeratinozyten; HUVEC: "human umbilical vein endothelial cells", menschliche Nabelschnurvenenendothelzellen.

Die Polymere der Vergleichsbeispiele 1 und 2 weisen sowohl für HKER als auch für HUVEC bereits bei sehr niedrigen Konzentrationen, d.h. einer IC₅₀ von etwa 0,01 % oder darunter, deutlich toxische Wirkung auf. Die von den Erfindern hergestellten neuen Polymere der Beispiele 1 und 3 zeigen im Vergleich dazu erst bei deutlich höheren Konzentrationen einen toxischen Effekt: Die IC₅₀ liegt für Beispiel 1 für beide Zelltypen bei rund 1 % und für Beispiel 3 im Bereich zwischen 0,05 % und 0,1 %. Die Toxizität der Vergleichsbeispiele wird mit dem Polymer aus Beispiel 3 somit erst bei der zumindest 5fachen Konzentration und mit jenem aus Beispiel 1 sogar erst in zumindest 100facher Konzentration erreicht. In diesem Test schnitt demnach das DAG-Derivat klar besser ab als das MAG-Polymer.

Die neuen Verbindungen weisen somit sehr gute bis ausgezeichnete Aktivität gegen diverseste Erreger auf - und das bei erheblich niedrigerer Toxizität als nach dem Stand der Technik bekannte Polyguanidin-Derivate.

## Patentansprüche

1. Polykondensationsprodukt von Aminoguanidin und/oder 1,3-Diaminoguanidin mit einem oder mehreren Diaminen, wobei das Polykondensationsprodukt ein Polyguanidin-Derivat der nachstehenden Formel (I) ist: 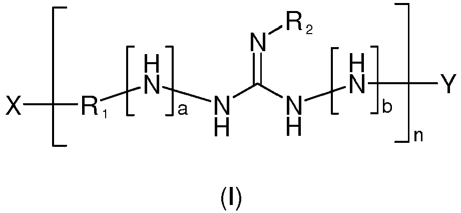 worin
X aus -NH₂, Aminoguanidino und 1,3-Diaminoguanidino ausgewählt ist;
Y aus -H und -R₁-NH₂ ausgewählt ist;
oder X und Y zusammen für eine chemische Bindung stehen, um eine zyklische Struktur zu ergeben;
R₁ aus zweiwertigen organischen Resten mit 2 bis 20 Kohlenstoffatomen ausgewählt ist, in denen gegebenenfalls ein oder mehrere Kohlenstoffatome durch O oder N ersetzt sind;
a und b jeweils 0 oder 1 sind,
wobei a+b ≠ 2 ist, wenn keine 1,3-Diaminoguanidin-Einheiten enthalten sind;
R₂ aus -H und -NH₂ ausgewählt ist, wobei
R₂-NH₂ ist, wenn a+b = 0 ist,
R₂ -H oder -NH₂ ist, wenn a+b = 1 ist und
R₂ -H ist, wenn a+b = 2 ist; und
n ≥ 2 ist;
oder ein Salz davon.

2. Polyguanidin-Derivat nach Anspruch 1, **dadurch gekennzeichnet, dass** R₁ aus Alkylenresten ausgewählt ist, in denen gegebenenfalls ein oder mehrere Kohlenstoffatome durch O oder N ersetzt sind.

3. Polyguanidin-Derivat nach Anspruch 2, **dadurch gekennzeichnet, dass** R₁ aus Resten der nachstehenden allgemeinen Formeln (II) bis (V) ausgewählt ist:
-(CH₂)_{c}-Z₁-(CH₂)_{d}-, (II)
-(CH₂)_{c}-Z₁-(CH₂)_{d}-Z₂-(CH₂)ₑ-, (III)
-(CH₂)_{c}-Z₁-(CH₂)_{d}-Z₂-(CH₂)ₑ-Z₃-(CH₂)_{f}-, (IV)
-(CH₂)_{c}-Z₁-(CH₂)_{d}-Z₂-(CH₂)ₑ-Z₃-(CH₂)_{f}-Z₄-(CH₂)_{g}-, (V)
worin Z₁ bis Z₄ jeweils unabhängig voneinander für ein aus O und N ausgewähltes Heteroatom stehen und die Indizes c bis g jeweils unabhängig voneinander für eine solche ganze Zahl im Bereich von 1 bis 12 stehen, dass die Gesamtanzahl der Atome des Rests R₁ 20 nicht überschreitet.

4. Polyguanidin-Derivat nach Anspruch 3, **dadurch gekennzeichnet, dass** innerhalb eines Rests R₁ alle Heteroatome Z entweder O oder N sind.

5. Polyguanidin-Derivat nach Anspruch 4, **dadurch gekennzeichnet, dass** R₁ für den zweiwertigen Rest eines Polyetherdiamins steht.

6. Polyguanidin-Derivat nach einem der Ansprüche 1 bis 5, **dadurch gekennzeichnet, dass** n = 2 bis 6 ist.

7. Polyguanidin-Derivat nach einem der Ansprüche 1 bis 6, **dadurch gekennzeichnet, dass** es als Säureadditionssalz in Form eines Hydrochlorids, Hydrobromids, Hydroiodids, Sulfats, Carbonats, Borats, Cyanats, Thiocyanats, Phosphats, Mesylats, Nitrats, Acetats, Benzoats, Lactats, Tartrats, Citrats, Maleats, Fumarats, eines Partialesters einer dieser Säuren, sofern sie di- oder höherfunktionell ist, oder eines Gemischs zweier oder mehrerer dieser Salze und/oder Partialester vorliegt

8. Verfahren zur Herstellung von Polyguanidin-Derivaten nach einem der Ansprüche 1 bis 7 durch Polykondensation eines Guanidin-Derivats oder eines Salzes davon mit einem Diamin, **dadurch gekennzeichnet, dass** Aminoguanidin oder 1,3-Diaminoguanidin oder ein Säureadditionssalz davon mit zumindest einem Diamin H₂N-R-NH₂ durch Erhitzen polykondensiert wird.

9. Verfahren nach Anspruch 8, **dadurch gekennzeichnet, dass** das zumindest eine Diamin in einem Überschuss von 3 bis 5 Mol-%, bezogen auf das Guanidin-Derivat, eingesetzt wird.

10. Verfahren nach Anspruch 8 oder 9, **dadurch gekennzeichnet, dass** ein Salz von Aminoguanidin oder 1,3-Diaminoguanidin zusammen mit dem zumindest einen Diamin zunächst auf eine erste, niedrigere Temperatur und dann auf eine zweite, höhere Temperatur erhitzt wird.

11. Verfahren nach Anspruch 10, **dadurch gekennzeichnet, dass** das Salz von Aminoguanidin oder 1,3-Diaminoguanidin zusammen mit dem zumindest einen Diamin zunächst auf 110-130 °C und dann auf 160-180 °C erhitzt wird.

12. Verfahren nach Anspruch 10 oder 11, **dadurch gekennzeichnet, dass** das Reaktionsgemisch 1 bis 3 h lang auf der ersten Temperatur und 1 bis 8 h lang auf der zweiten Temperatur gehalten wird.

13. Verfahren nach einem der Ansprüche 8 bis 12, **dadurch gekennzeichnet, dass** das erhaltene Polyguanidin-Derivat durch Lösen in etwa der 3- bis 10fachen Menge Wasser gereinigt wird.

14. Polyguanidin-Derivat nach einem der Ansprüche 1 bis 7 oder hergestellt durch ein Verfahren nach einem der Ansprüche 8 bis 13 zur Verwendung im human- und veterinärmedizinischen Bereich zur Bekämpfung von bakteriellen, fungalen und viralen Infektionen und deren Folgeerscheinungen, als Pestizid und Desinfektionsmittel im Agrar- und Umweltbereich, allgemein als Desinfektionsmittel (Biozid) zur Keimreduktion und -vernichtung, als Antiparasitikum, als Zusatz zur Stabilisierung (Sterilisation) von Produkten oder auch als Vernebelungssubstanz in gelöster Form zur Kalt-/Nass-Vernebelung, Mikronisierung oder Dampfsterilisation.

15. Polyguanidin-Derivat nach einem der Ansprüche 1 bis 7 oder hergestellt durch ein Verfahren nach einem der Ansprüche 8 bis 13 zur Verwendung gemäß Anspruch 14, **dadurch gekennzeichnet, dass** eine wirksame Menge des Polyguanidin-Derivats als Lösung in der 3- bis 10fachen Menge an Wasser vorliegt.

## Claims

1. A polycondensation product of aminoguanidine and/or 1,3-diaminoguanidine with one or more diamines, wherein said polycondensation product is a polyguanidine derivative of the following formula (I): 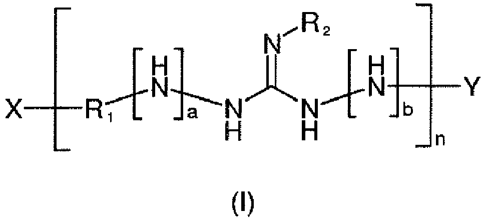 wherein
X is selected from -NH₂, aminoguanidino, and 1,3-diaminoguanidino;
Y is selected from -H and -R₁-NH₂;
or X and Y taken together represent a chemical bond, which results in a cyclic structure;
R₁ is selected from bivalent organic residues having 2 to 20 carbon atoms, wherein one or more carbon atoms are optionally replaced by O or N;
a and b are each 0 or 1,
wherein a+b ≠ 2 if no 1,3-diaminoguanidine units are contained;
R₂ is selected from -H and -NH₂, wherein
if a+b = 0, R₂ is -NH₂,
if a+b = 1, R₂ is -H and -NH₂, and
if a+b = 2, R₂ is -H; and
n is ≥ 2;
or a salt thereof.

2. The polyguanidine derivative according to claim 1, **characterized in that** R₁ is selected from alkylene residues in which one or more carbon atoms are optionally replaced by O or N.

3. The polyguanidine derivative according to claim 2, **characterized in that** R₁ is selected from residues of the following formulae (II) to (V):
-(CH₂)_{c}-Z₁-(CH₂)_{d}-, (II)
-(CH₂)_{c}-Z₁-(CH₂)_{d}-Z₂-(CH₂)ₑ-, (III)
-(CH₂)_{c}-Z₁-(CH₂)_{d}-Z₂-(CH₂)ₑ-Z₃-(CH₂)_{f}-, (IV)
-(CH₂)_{c}-Z₁-(CH₂)_{d}-Z₂-(CH₂)ₑ-Z₃-(CH₂)ₜ-Z₄-(CH₂)_{g}-, (V)
wherein Z₁ to Z₄ each independently represent a heteratom selected from O and N and the subscripts c to g each independently represent such an integer in the range from 1 to 12 that the total number of atoms of the residue R₁ does not exceed 20.

4. The polyguanidine derivative according to claim 3, **characterized in that** all heteroatoms Z within one residue R₁ are either O or N.

5. The polyguanidine derivative according to claim 4, **characterized in that** R₁ represents a bivalent polyetherdiamine radical.

6. The polyguanidine derivative according to any of the claims 1 to 5, **characterized in that** n is 2 to 6.

7. The polyguanidine derivative according to any of the claims 1 to 6, **characterized in that** it is an acid addition salt in the form of a hydrochloride, hydrobromide, hydroiodide, sulphate, carbonate, borate, cyanate, thiocyanate, phosphate, mesylate, nitrate, acetate, benzoate, lactate, tartrate, citrate, maleate, fumarate, or a partial ester of one of these acids if they are bifunctional or of a higher functionality, or a mixture of two or more of these salts and/or partial esters.

8. A process for producing the polyguanidine derivatives according to any one of the claims 1 to 7 by polycondensation of a guanidine derivative or a salt thereof with a diamine, **characterized in that** aminoguanidine or 1,3-diaminoguanidine or an acid addition salt thereof is polycondensated with at least one diamine H₂N-R-NH₂ by heating.

9. The process according to claim 8, **characterized in that** said at least one diamine is used in an excess of 3 to 5 mol% in relation to the guanidine derivative.

10. The process according to claim 8 or 9, **characterized in that** a salt of aminoguanidine or 1,3-diaminoguanidine is heated together with said at least one diamine, at first to a first lower temperature and then to a second higher temperature.

11. The process according to claim 10, **characterized in that** the salt of aminoguanidine or 1,3-diaminoguanidine is heated together with said at least one diamine, at first to a temperature of 110 to 130 °C and then to a temperature of 160 to 180 °C.

12. The process according to claim 10 or 11, **characterized in that** the reaction mixture is maintained at the first temperature for 1 to 3 hours and at the second temperature for 1 to 8 hours.

13. The process according to any one of the claims 8 to 12, **characterized in that** the obtained polyguanidine derivative is purified by by dissolution in approximately the 3- to 10-fold amount of water.

14. The polyguanidine derivative according to any one of the claims 1 to 7 or produced by the process according to any one of the claims 8 to 13 for use in the human and veterinary medical fields for antagonizing bacterial, fungal and viral infections and their aftereffects, as a pesticide and disinfectant in the agricultural and environmental fields, generally as a disinfectant (biocide) for reducing and eliminating germs, as an antiparasitic, as a supplement for stabilizing (sterilizing) products, or as a nebulization substance in a dissolved form for cold/wet nebulization, micronization and vapor sterilization.

15. The polyguanidine derivative according to any one of the claims 1 to 7 or produced by the method according to any one of the claims 8 to 13 for use according to claim 14, **characterized in that** an effective amount of the polyguanidine derivative is present as a solution in the 3- to 10-fold amount of water.

## Revendications

1. Produit de polycondensation de l'aminoguanidine et/ou de l'1,3-diaminoguanidine avec une ou plusieurs diamines, le produit de polycondensation étant un dérivé de la polyguanidine de formule (I) ci-dessus: 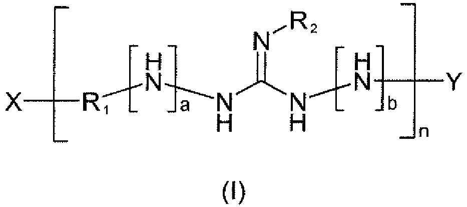 dans laquelle
X est choisi parmi -NH₂, aminoguanidino et 1,3-diaminoguanidino;
Y est choisi parmi -H et -R₁-NH₂; ou
X et Y représentent ensemble une liaison chimique pour former une structure cyclique;
R₁ est choisi parmi des radicaux organiques divalents ayant 2 à 20 atomes de carbone, dans lesquels éventuellement un ou deux atomes de carbone sont substitués par O ou N;
chacun de a et b est 0 ou 1,
a + b ≠ 2, si aucune unité de l'1,3-diamunoguanidine est présente;
R₂ est choisi parmi -H et -NH₂,
R2 étant -NH₂, si a+b=0,
R₂ étant -H ou -NH₂, si a+b=1, et
R₂ étant -H, si a+b=2; et
n est ≥ 2;
ou un sel de celui-ci.

2. Dérivé de la polyguanidine selon la revendication 1, **caractérisé en ce que** R₁ est choisi parmi des radicaux alkylènes, dans lesquels un ou plusieurs atomes de carbone sont éventuellement substitués par O ou N.

3. Dérivé de la polyguanidine selon la revendication 2, **caractérisé en ce que** R₁ est choisi parmi des radicaux des formules générales (II) à (V) ci-dessus:
-(CH₂)_{c}-Z₁-(CH₂)_{d}-, (II)
-(CH₂)_{c}-Z₁-(CH₂)_{d}-Z₂-(CH₂)ₑ-, (III)
(CH₂),-Z₁-(CH₂)_{d}-Z₂-(CH₂)ₑ-Z₃-(CH₂)_{f}, (IV)
-(CH₂)_{c}-Z₁-(CH₂)_{d}-Z₂-(CH₂)ₑ-Z₃-(CH₂)_{f}-Z₄-(CH₂)_{g}-, (V)
dans lesquelles chacun de Z₁ à Z₄ représente indépendamment un hétéroatome choisi parmi O et N, et chacun des indices c à g représente indépendamment un nombre entier compris entre 1 et 12 de sorte que le total des atomes du radical R₁ n'est pas supérieur à 20.

4. Dérivé de la polyguanidine selon la revendication 3, **caractérisé en ce que** tous les hétéroatomes Z dans un radical R₁ sont soit O soit N.

5. Dérivé de la polyguanidine selon la revendication 4, **caractérisé en ce que** R₁ représente le radical divalent d'une polyétherdiamine.

6. Dérivé de la polyguanidine selon l'une quelconque des revendications 1 à 5, **caractérisé en ce que** n = 2 à 6.

7. Dérivé de la polyguanidine selon l'une quelconque des revendications 1 à 6, **caractérisé en ce qu'**il est présent comme sel d'addition d'acide sous forme d'hydrochlorure, hydrobromide, hydroiodide, sulfate, carbonate, borate, cyanate, thiocyanate, phosphate, mesylate, nitrate, acétate, benzoate, lactate, tartrate, citrate, maléate, fumarate, ester partiel d'un de ces acides, à condition qu'il soit bifonctionnel ou de fonctionnalité supérieure, ou d'un mélange de deux ou plusieurs de ces sels et/ou esters partiels.

8. Procédé pour la production de dérivés de la polyguanidine selon l'une quelconque des revendications 1 à 7 par polycondensation d'un dérivé de la guanidine ou d'un sel de celui-ci avec une diamine, **caractérisé en ce que** l'aminoguanidine ou 1'1,3-diaminoguanidine ou un sel d'addition d'acide de celle-ci est polycondensé(e) par réchauffement avec au moins une diamine H₂N-R-NH₂.

9. Procédé selon la revendication 8, **caractérisé en ce que** l'au moins une diamine est utilisée en excès de 3 à 5 % en moles par rapport au dérivé de la guanidine.

10. Procédé selon la revendication 8 ou 9, **caractérisé en ce qu'**un sel de l'aminoguanidine ou de l'1,3-diaminoguanidine est réchauffée avec l'au moins une diamine, d'abord à une première température, inférieure, et ensuite à une deuxième température, supérieure.

11. Procédé selon la revendication 10, **caractérisé en ce que** le sel de l'aminoguanidine ou de l'1,3-diaminoguanidine est réchauffé avec l'au moins une diamine, d'abord à 110-130 °C et ensuite à 160-180 °C.

12. Procédé selon la revendication 10 ou 11, **caractérisé en ce que** le mélange de réaction est maintenu à la première température pendant 1 à 3h et à la deuxième température pendant 1 à 8h.

13. Procédé selon l'une quelconque des revendications 8 à 12, **caractérisé en ce que** le dérivé de la polyguanidine obtenu est purifié par dissolution dans une quantité d'eau correspondant à 3 à 10 fois sa quantité.

14. Dérivé de la polyguanidine selon l'une quelconque des revendications 1 à 7 ou obtenu par un procédé selon l'une quelconque des revendications 8 à 13 pour l'utilisation dans le domaine de la médecine humaine ou vétérinaire pour lutter contre les infections bactériennes, fongiques et virales et leurs conséquences, comme pesticide et désinfectant dans le domaine agraire et environnemental, comme désinfectant (biocide) pour la réduction et destruction de germes en général, comme parasiticide, comme additif pour la stabilisation (stérilisation) de produits ou encore, sous forme dissoute, comme substance de nébulisation pour la nébulisation à froid/à chaud, micronisation ou stérilisation à vapeur.

15. Dérivé de la polyguanidine selon l'une quelconque des revendications 1 à 7 ou obtenu par un procédé selon l'une quelconque des revendications 8 à 13 pour l'utilisation selon la revendication 14, **caractérisé en ce qu'**une quantité efficace du dérivé de la polyguanidine est présente sous forme de solution dans une quantité d'eau correspondant à 3 à 10 fois sa quantité.
